# EUROPEAN PATENT SPECIFICATION

(11) **EP 0 608 592 B1**
(45) Date of publication and mention of the grant of the patent: **05.08.1998**
(21) Application number: 93300548.0
(22) Date of filing: 26.01.1993
(51) Int. Cl.: A61B 17/58, B25B 25/00

(54) **Sternum banding assembly**
Cerclagegerät zum Schliessen des Brustbeins
Assemblage de cerclage pour fermer le sternum

(43) Date of publication of application: 03.08.1994
(73) Proprietor: STONY BROOK SURGICAL INNOVATIONS, INC., Northport, New York 11763 (US)
(72) Inventor: Blasnik, William, Fort Lee, New Jersey 07024 (US)
(74) Representative: Allam, Peter Clerk

(56) References cited:
- DE-A- 4 021 246
- US-A- 4 667 662
- US-A- 4 813 416

## Description

The present invention relates to an assembly for banding the sternum for use with a hand-held clamp. A sternum banding assembly is used for clamping and closing the split halves of the sternum as part of surgery which involves a median sternotomy, such as open heart surgery.

For proper healing of the split sternum, the surgically opened faces must be approximated, compressed and held together rigidly. This task is complicated by the physiological role of the sternum. The sternum is a functional component of the thoracic cage and, with the costal cartilages, serves as the hinge for the "bucket handle" action of the ribs during respiration. The incessant motion of the rib cage transmits continuous stresses across the sternum. Any method of closing a split sternum must be able to maintain compression and rigidity across the closure in the face of these constant stresses.

One technique used in the past involves closing the sternum with a plurality of spaced stainless steel wires. Five or more 20 gauge stainless steel wires are placed either parasternally (around the sternum) or transternally (through the sternum) using a large, swaged-on cutting needle. The needles are cut off and the sternal halves are approximated by twisting the wires. Finally the wires are cut short and the ends are tucked into the adjacent tissue.

While this is a useful surgical technique for closing the sternum, there are certain problems associated with this procedure. The wires are difficult to place and if to be placed transternally, the needle must be driven through the sternum, a very difficult task. The internal mammary artery is subject to being injured during the procedure. Also, the sharp wires often cause cutting of surgical gloves and may injure the surgeon. Twisting the wires while tightening may produce torsional stresses and may even severely weaken or fracture the wires. The stresses imparted by respiratory motion of the chest cage can further fatigue or break the wires. The wires may also slice through thin or osteoporotic bone. Hence closure of the sternum with wires is a slow and tedious if not dangerous technique.

To overcome the drawbacks of conventional wire banding, it has been proposed to use a board in front of the sternum through which the wires pass. Others have suggested replacing the wires with various types of clamps or closures.

Presently, the best system seems to be to employ flat flexible stainless steel bands instead of wires. Each band may be formed integrally with a surgical needle at one end and a locking member at the other end. One type of such band is disclosed in U.S. Patent No. 4,535,764 to Ebert. However, Ebert's locking member requires that the band be threaded through an opening in the locking member and bent back over the member and threaded through two other openings, a complicated and time consuming task. Another variation is disclosed in U.S. Patent No. 4,730,615 to Sutherland. Sutherland uses a polymer coated metal band with an upstanding head. Adjacent the head is a serrated spine portion which is locked by a metal finger on a channel in the head.

A significant improvement over the assemblies taught by Sutherland and Ebert is the bonding or banding assembly disclosed in U.S. Patent No. 4,813,416, issued to William Blasnik and Stanley Pollak. That banding assembly includes a locking member in the form of a buckle. The buckle has a loop segment under the saddle. The segment terminates in a spring tooth adapted to engage an opening in the band when the band is pulled through a channel. The channel is defined by "C" shaped flanges extending upwardly from either side of the saddle.

In spite of the success of the band disclosed in U.S. Patent No. 4,813,416, it too has certain drawbacks. The buckle is not flat because of the loop segment. However, the loop cannot be eliminated because as the band is pulled through the buckle, the buckle must be held by a clamp which engages the loop. Threading the band through the "C" flanges is sometimes difficult because of the shape of the buckle and rigidity of the flanges. Once the band is tightened, it must be trimmed. This may result in a tail with one or more sharp edges which are exposed.

An embodiment of the present invention is described below. It is an improvement over the band of U.S. Patent No. 4,813,416 in that the structure of the buckle has been substantially revised to make it flat by eliminating the loop segment entirely and by providing new members in the form of upstanding ears with clamp receiving openings to permit the buckle to be held firmly as the band is tightened. The ears serve a second function. They are spaced in relation to the band width such that once the band is pulled through the channel, tightened, locked and trimmed, the remaining tail can be bent back over the buckle and snap-fitted between the ears to a position proximate the buckle floor such that it is safely tucked away. Crimping the ears over the tail insures that no sharp edges are exposed, permanently locks the band and at the same time, reduces the profile of the buckle.

Threading the band into the channel in the buckle is facilitated through the use of a guide member situated on the band. More particularly, the guide member takes the form of a dome-like element situated on the surface of the band adjacent a narrowed portion. The element and the narrow band portion cooperate with the "C" shaped channel defining flanges to assist in threading the band through the channel.

The preferred embodiment of the present invention provides an improved sternum banding assembly which includes a buckle designed to be engaged by a clamp to facilitate tightening of the band but which has a low profile. The assembly eliminates any exposed sharp edges by enclosing the trimmed tail within the buckle. Furthermore insertion of the band into the channel in the buckle is facilitated by a dome-shaped guide element.

Broadly stated the above-mentioned U.S. patent 4,813,416 discloses an assembly for banding the sternum for use with a hand-held clamp of the kind set out in the preambles to Claims 1 and 11.

As discussed above, in U.S. patent 4,813,416, the means for engagement by the clamp in this kind of assembly comprises a loop segment under the saddle of the buckle.

According to the present invention the assembly is characterized as set forth in Claims 1 and 11.

In the proposed use of the assembly the end section of the band, after the band is drawn into the channel and locked into position by the locking means, is bent back over the surface-cooperating means (also referred to as channel-defining means). The engagement means comprises means for retaining and securing the bent end section.

The portion of the band that is to be retained has a given width. The upstanding elements are normally spaced apart, at their closest point, a distance which is slightly less than this given width. The retained portion preferably includes a trimmed end of the band.

In the described embodiment the band has first and second openings. Means are provided for attaching the buckle to the band. The attaching means comprises first and second elements extending from the buckle and adapted to be received in the first and second band openings, respectively.

The band to be described also includes a portion proximate the one end. That portion includes a decreased width section and guide member, preferably in the form of a substantially dome-shaped guide element, for assisting in passing the band through the channel in the buckle.

Another aspect of the invention is a sternum banding assembly for use with a hand-held clamp as set forth in Claim 11.

The hand-held clamp used with the assembly is preferably one having a pair of clamp arms moveable between proximate and remote positions. The clamp may be a conventional towel clamp. The opening in each element (also referred to later as an "ear") is adapted to receive a different one of the clamp arms. When the clamp arms are moved from the remote position to the proximate position, the buckle is engaged and retained by the clamp allowing the band to be drawn through the channel to the locking position to form the desired loop.

A method for banding a sternum with a banding assembly according to the invention is as follows. The banding assembly referred to is the initial assembly having the surgical needle fixed to one end. It is assumed that the clamp is of the type mentioned having arms moveable between remote and proximate positions. The method includes the steps of threading the needle and band around or through the sternum parts. The needle is then removed from the band end. The band end is guided through the channel defining elements in the buckle. The buckle is retained with the clamp by moving the arms of the clamp from the remote position to the proximate position and engaging the openings in the upstanding ears. The band is tightened by pulling it through the channel as the buckle is retained by the clamp, until the band is locked by the locking member. The excess band is cut off, leaving a tail. The band tail is bent over the channel-defining elements and placed between the upstanding ears. The ears are crimped over the band. They overlie the trimmed end of the band.

The upstanding ears are spaced apart a distance slightly less than the width of the tail portion of the band retained by the ears. The step of placing the band tail between the upstanding ears comprises the step of snap-fitting the band tail between the ears.

A sternum banding assembly in accord with the present invention and a method of using it will now be described with reference to the embodiment illustrated in the accompanying drawings, wherein like numerals refer to like parts, and in which:
Fig. 1 is a top plan view of the banding assembly of the present invention;
Fig. 2 is a side view of the banding assembly of Fig. 1;
Fig. 3 is an enlarged top plan view of the band guide member;

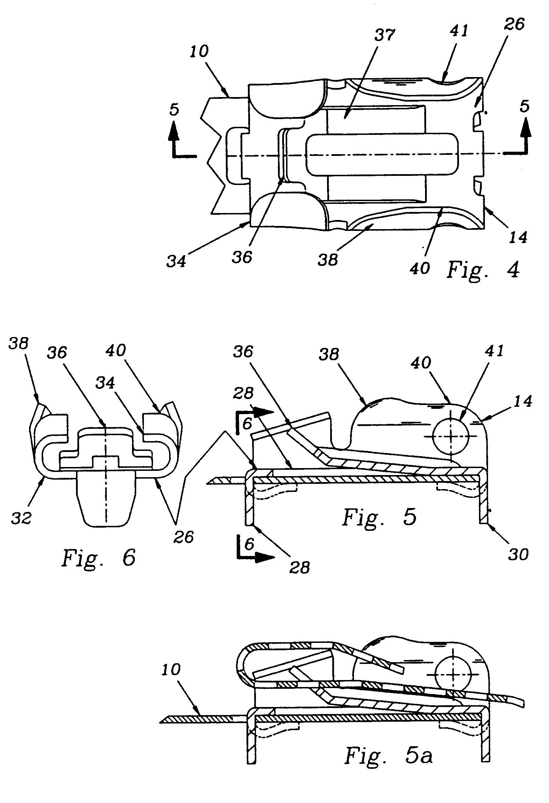

Fig. 4 is an enlarged top plan view of the buckle;
Fig. 5 is a side cutaway view of the buckle of Fig. 4, taken along line 5-5;
Fig. 5(a) is a side cutaway view of the buckle of Fig. 4, showing the band tail after crimping;
Fig. 6 is a front view of the buckle of Fig. 4, taken along line 6-6 thereof;

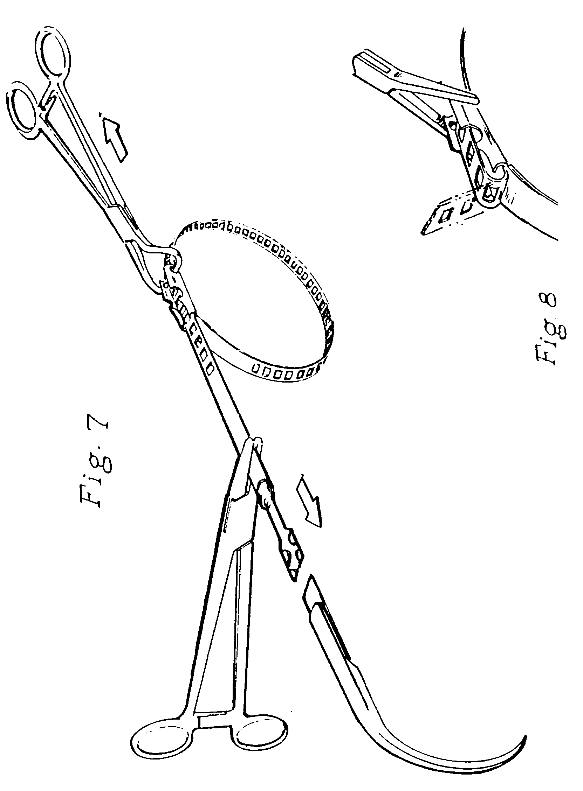

Fig. 7 is a perspective view of the banding assembly showing the buckle retained by a towel clamp;
Fig. 8 is a perspective view of the buckle showing the band tail being placed and the ears being crimped;
Figs. 9(a)-9(g) collectively illustrate the steps employed to band a split sternum with the banding assembly of the present invention.

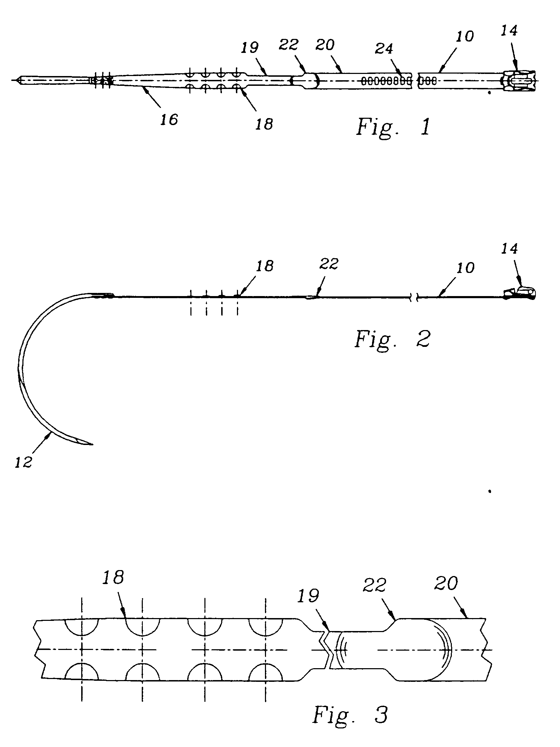

As seen in Figures 1 and 2, the banding assembly includes an elongated, thin and relatively wide flat band 10, preferably made of stainless steel. A surgical needle 12 extends from one end of band 10. Needle 12 is preferably integral with the band but may be a separate part riveted or otherwise affixed to the band. Mounted proximate the other end of band 10 is a buckle 14.

Needle 12 is curved to facilitate insertion from one parasternal location, under the sternum halves and then outwardly at an opposite parasternal location. Adjacent needle 12 is a band section 16 of gradually increasing width. Section 16 has protrusions or ripples 18 on the top surface. Ripples 18 serve to gently expand the opening in the bone to permit the band to pass through, even when kinked or distorted.

Next to ripple section 16 is a relatively narrow section 19 which widens to the main band section 20. Situated proximate the connection between sections 18 and 20 is a dome-shaped guide member 22 which extends above the band surface, preferably to the plane of the top surface of ripples 18. Member 22 facilitates entry of the main section 20 of the band into the channel in buckle 14. Main section 20 has a plurality of spaced slots 24. Slots 24 are adapted to be engaged by the locking mechanism 37 in the buckle.

The buckle 14 comprises a substantially planar floor 26 having downwardly extending tabs 28, 30 at the forward and rear edge, respectively. Tabs 28, 30 are adapted to extend through spaced openings near the end of band section 20. Tabs 28, 30 are bent inwardly to secure buckle 14 to band 10.

A pair of oppositely oriented spaced "C" shaped flanges 32 extend upwardly from opposite sides of floor 26, beginning at the forward end of the buckle and extending toward the rear for approximately one third of the length of the buckle. Flanges 32 are relatively thick and rigid and define the channel into which band 10 will be received. The inwardly extending top portions 34 of the flanges are inclined upwardly, away from floor 26. Thus, the channel is wedge shaped, being smallest at its mouth and gradually becoming larger toward the rear of the buckle.

Extending upwardly from floor 26, toward the plane of top portions 34, is a locking tooth 36. Tooth 36 is adapted to be received within one of the slots 24 in band 10 to lock the band in position within the channel defined by flanges 32.

Also extending from floor 26, at either side thereof, and substantially aligned with flanges 32 are a pair of upstanding ears 38. Ears 38 are slightly curved toward each other such that the top edges 40 of the ears are the points which are closest together. Edges 40 are spaced apart by a dimension which is slightly less than the width of band section 20. The flexibility of the stainless steel material of which ears 38 are made permits a section of the band to "snap-fit" between the ears as explained below.

Ears 38 are each provided with openings 41, shaped and sized to accept the moveable arms of a conventional towel clamp. Thus, the buckle can be engaged and retained by the arms of a towel clamp as the band is pulled through the channel and tightened into the locking position such that tooth 36 protrudes into one of the slots 24 in the band.

Ears 38 serve the additional purpose of permanently retaining the tail 42 of the band, after it has been trimmed, in close face to face relation with spring element 37, in a way which covers any sharp edges of the band which may result from trimming the tail. As is explained below, once the band is locked in position in the channel, it is trimmed to have a tail with a length about as long as the buckle. The tail is bent back over flanges 32, toward the rear of the buckle, and snap fits between ears 38. Ears 38 are then crimped inwardly, covering the edges of the tail band. In this way, sharp or jagged edges are protected and a low profile is achieved.

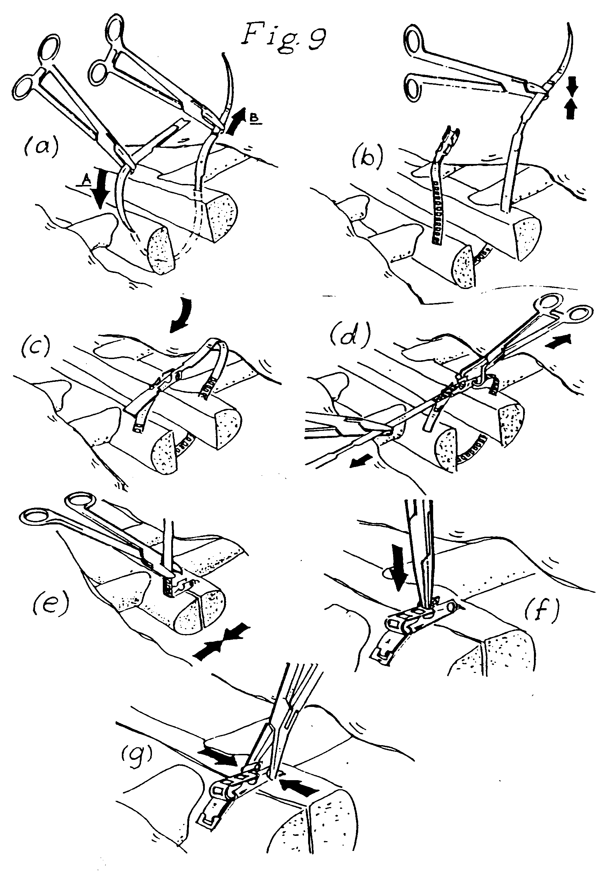

The method of using the banding assembly of the present invention is illustrated in Figs. 9(a)-9(g) which show the various operations involved. When the sternum is ready to be closed, needle 12 is threaded through or around the sternum sections, as shown in Fig. 9(a). Hemostats or needle holders are used to manipulate the band and needle. Once the band is in place (Fig. 9(b)), the needle 12 is cut off, about an inch from narrow section 18. The narrow section 18 is then inserted between flanges 32 and dome-shaped member 22 guides the main section 20 of the band into the channel (Fig. 9(c)). A clamp is used to pull the band tight while the buckle is retained by a towel clamp whose arms are received in openings 41 (Fig. 9(d)). Once the band is in position, tooth 36 lodges in one of the slots 24 urged upwardly by spring element 37, to lock the band within the channel. The band is bent upwardly, 90° from floor 26 of the buckle and trimmed to form a tail 42, approximately 4 slots long (Fig. 9(e)).

Tail 42 is bent back over the buckle and particularly over flanges 32 and "snap fit" between ears 38 as illustrated in Fig. 9(f). Ears 38 are crimped to overlap the edges of the tail. (Fig. 9(g). In this way, the tail is permanently retained within the buckle and no sharp edges are exposed.

The entire procedure can be performed quickly and efficiently, using only conventional operating room instruments. The resulting structure has a low profile, with no protruding parts or sharp edges. However, the band is reliably locked by the tooth and held securely by the folded tail 42.

It should now be appreciated that there has been described an improved sternum banding assembly in which spaced upstanding ears, aligned with the channel defining flanges serve as a means for engagement with a towel clamp to retain the buckle as the band is tightened and to capture the tail in a manner which covers any sharp edges which would otherwise be exposed. In addition, a dome-like guide member is employed to facilitate entrance of the main section of the band into the channel in the buckle.

## Claims

1. An assembly for banding the sternum for use with a hand-held clamp, comprising an elongate flexible band (10), a needle (12) at one end of the band (10) and a buckle (14) proximate the other end of the band (10), the buckle comprising a surface (26) and means (34) co-operating with said surface (26) to define a channel in which the band is receivable, means (36 and 37) for locking a portion (24) of the band in said channel to secure the band in a sternum banding loop, and means (38) for engagement by the clamp to permit the buckle (14) to be retained by the clamp as said band portion (24) is locked into said channel by said locking means (36 and 37), characterized in that:
said clamp engagement means (38) comprises a pair of upstanding elements aligned with said channel, each element having a respective opening (41) therein to receive a respective part of the clamp.

2. An assembly as claimed in Claim 1 in which said engagement means (38) comprises retaining means (40) in which a further portion of said band is retainable after bending back over said surface-cooperating means (34) the band extending from said channel.

3. An assembly as claimed in Claim 2 in which said retaining means (40) comprises portions of said upstanding elements (38).

4. An assembly as claimed in Claim 1 in which said channel is located at the forward end of the buckle and said upstanding elements of said engagement means (38) are rearward of the channel and comprise retaining means (40) for retaining a further portion of the band (10) extending from said channel having bent the band back over said surface-cooperating means (34).

5. An assembly as claimed in Claim 2, 3 or 4 wherein said further portion of the band has a given width and wherein said retaining means (40) are normally spaced apart, at their closest point, a distance which is slightly less than said given width.

6. An assembly as claimed in any one of Claims 1 to 5 in which the band comprises a guide member (22) for guiding the band through said channel in forming said loop.

7. An assembly as claimed in Claim 6 in which said guide-member (22) is dome-shaped.

8. An assembly as claimed in Claim 6 in which said surface-cooperating means (34) defines with said surface (26) a wedge-shaped channel (Fig. 5) narrowing in the direction toward the forward end of the buckle (14).

9. An assembly as claimed in Claim 6 or 7 wherein said band comprises a section (19) of decreased width adjacent said dome-shaped guide member (22) at the side thereof toward said one end of the band (10).

10. An assembly as claimed in any preceding claim wherein said band (10) has first and second openings proximate said other end thereof, and said buckle (14) comprises first (28) and second (30) elements extending from said buckle (14) and received in said first and second band opening, respectively, to attach the buckle (14) to the band.

11. A sternum banding assembly for use with a hand-held clamp, comprising an elongate flexible band formed to provide a loop (10), one end of said band formed by trimming, and a buckle (14) proximate the other end of the band (10), the buckle (14) comprising a surface (26) and means (34) cooperating with said surface (26) to define a channel in which a portion (24) of the band (10) is received, said loop extending from said other end to said portion (24) of the band, means (36 and 37) for locking said band portion (24) in the channel, and means (38) for engagement by the clamp to permit the buckle (14) to be retained by the clamp as said band portion (24) is locked into the buckle (14) by said locking means (36 and 37), characterized in that:
said clamp engagement means (38) comprises a pair of upstanding elements aligned with said channel, each element having a respective opening therein (41) to receive a respective part of the clamp.

12. An assembly as claimed in Claim 11 in which said engagement means (38) comprises retaining means (40) into which a further portion of the band including said trimmed end is retained, said further portion and the portion (24) of the band locked in said channel being connected by a portion of the band (10) bent back over said surface-cooperating means (34).

13. An assembly as claimed in Claim 12 in which said retaining means (40) comprises portions of said upstanding elements (38).

14. An assembly as claimed in Claim 11 in which said channel is toward the forward end of the buckle (14) and said upstanding elements of said engagement means (38) are located rearwardly of said channel and comprise retaining means (40) retaining said further portion of the band and overlying said trimmed end.

15. An assembly as claimed in Claim 12, 13 or 14 in which said retaining means (40) are spaced apart, at their closest point, a distance less than the width of said further portion of the band.

16. An assembly as claimed in any one of Claims 11 to 15 in which said surface-cooperating means (34) defines with said surface (26) a wedge-shaped channel (Fig. 5) narrowing toward the forward end of the buckle (14).

17. An assembly as claimed in any one of Claims 11 to 16 wherein said band (10) has first and second openings proximate said other end thereof, and said buckle (14) comprises first (28) and second (30) elements extending from said buckle (14) and received in said first and second band opening, respectively, to attach the buckle to the band (10).

## Patentansprüche

1. Baugruppe zum Bandagieren des Sternums für den Gebrauch mit einer handgehaltenen Klammer, umfassend ein längliches flexibles Band (10), eine Nadel (12) an einem Ende des Bandes (10) und eine Schnalle (14) in der Nähe des anderen Endes des Bandes (10), wobei die Schnalle folgendes umfaßt: eine Fläche (26) und ein Mittel (34), das mit der genannten Fläche (26) zusammenwirkt, um einen Kanal zu definieren, in dem das Band aufgenommen werden kann, Mittel (36 und 37) zum Arretieren eines Abschnitts (24) des Bandes in dem genannten Kanal, um das Band in einer Sternumbandageschlinge zu sichern, und ein Mittel (38) für den Eingriff mit der Klammer, damit die Schnalle (14) von der Klammer festgehalten werden kann, wenn der genannte Bandabschnitt (24) in dem genannten Kanal durch das genannte Arretiermittel (36 und 38) arretiert wird, dadurch gekennzeichnet, daß:
das genannte Klammereingriffmittel (38) ein Paar aufrechtstehender Bestandteile umfaßt, die auf den Kanal ausgerichtet sind, wobei jeder Bestandteil jeweils über eine Öffnung (41) verfügt, um einen jeweiligen Teil der Klammer aufzunehmen.

2. Baugruppe nach Anspruch 1, bei der das genannte Eingriffmittel (38) Haltemittel (40) umfaßt, in denen ein weiterer Abschnitt des genannten Bandes festgehalten werden kann, nachdem das von dem genannten Kanal wegverlaufende Band zurück über das genannte, mit der Fläche zusammenwirkende Mittel (34) gebogen wurde.

3. Baugruppe anch Anspruch 2, bei der die genannten Haltemittel (40) Abschnitte der genannten aufrechtstehenden Bestandteile (38) umfassen.

4. Baugruppe nach Anspruch 1, bei der sich der genannte Kanal am vorderen Ende der Schnalle befindet und die genannten aufrechtstehenden Bestandteile des genannten Eingriffmittels (38) sich hinter dem Kanal befinden und Haltemittel (40) umfassen, um einen weiteren Abschnitt des von dem Kanal wegverlaufenden Bandes (10) festzuhalten, wobei das Band zurück über das genannte, mit der Fläche zusammenwirkende Mittel (34) gebogen ist.

5. Baugruppe nach Anspruch 2, 3 oder 4, bei der der genannte weitere Abschnitt des Bandes eine vorgegebene Breite hat und bei der die genannten Haltemittel (40) an ihrer engsten Stelle normalerweise über eine Distanz voneinander beabstandet sind, die etwas kürzer ist als die genannte vorgegebene Breite.

6. Baugruppe nach einem der Ansprüche 1 bis 5, bei der das Band ein Führungsglied (22) zum Führen des Bandes durch den genannten Kanal beim Formen der genannten Schlinge umfaßt.

7. Baugruppe nach Anspruch 6, bei der das genannte Führungsglied (22) kuppelförmig ist.

8. Baugruppe nach Anspruch 6, bei der das genannte, mit der Fläche zusammenwirkende Mittel (34) mit der genannten Fläche (26) einen keilförmigen Kanal definiert (Fig. 5), der in Richtung auf das vordere Ende der Schnalle (14) enger wird.

9. Baugruppe nach Anspruch 6 oder 7, bei der das genannte Band einen Bereich (19) reduzierter Breite neben dem genannten kuppelförmigen Führungsglied (22) auf seiner Seite in Richtung auf das genannte eine Ende des Bandes (10) umfaßt.

10. Baugruppe nach einem der vorherigen Ansprüche, bei der das genannte Band (10) über eine erste und eine zweite Öffnung in der Nähe seines genannten anderen Endes verfügt, und die genannte Schnalle (14) ein erstes (28) und ein zweites (30) Bestandteil umfaßt, die von der genannten Schnalle (14) wegverlaufen und jeweils in der genannten ersten und der zweiten Bandöffnung aufgenommen werden, um die Schnalle (14) an dem Band zu befestigen.

11. Sternumbandagebaugruppe für den Gebrauch mit einer handgehaltenen Klammer, umfassend ein längliches flexibles Band, das so geformt ist, daß es eine Schlinge (10) bildet, wobei ein Ende des genannten Bandes durch Beschneiden geformt ist, und eine Schnalle (14) in der Nähe des anderen Endes des Bandes (10), wobei die Schnalle (14) folgendes umfaßt: eine Fläche (26) und ein Mittel (34), das mit der genannten Fläche (26) zusammenwirkt, um einen Kanal zu definieren, in dem ein Abschnitt (24) des Bandes (10) aufgenommen wird, wobei die genannte Schlinge von dem genannten anderen Ende zu dem genannten Abschnitt (24) des Bandes verläuft, Mittel (36 und 37) zum Arretieren des genannten Bandabschnitts (24) in dem Kanal, und Mittel (38) für einen Eingriff mit der Klammer, damit die Schnalle (14) von der Klammer festgehalten werden kann, wenn der genannte Bandabschnitt (24) in der Schnalle (14) von dem genannten Arretiermittel (36 und 37) arretiert wird, dadurch gekennzeichnet, daß:
das genannte Klammereingriffmittel (38) ein Paar aufrechtstehender Bestandteile umfaßt, die auf den genannten Kanal ausgerichtet sind, wobei jedes Bestandteil jeweils über eine Öffnung (41) verfügt, um einen jeweiligen Teil der Klammer aufzunehmen.

12. Baugruppe nach Anspruch 11, bei der das genannte Eingriffmittel (38) Haltemittel (40) umfaßt, in denen ein weiterer Abschnitt des Bandes gehalten wird, der das genannte beschnittene Ende beinhaltet, wobei der genannte weitere Abschnitt und der Abschnitt (24) des Bandes, der in dem genannten Kanal arretiert ist, durch einen Abschnitt des Bandes (10) verbunden werden, der zurück über das genannte, mit der Fläche zusammenwirkende Mittel (34) gebogen wird.

13. Baugruppe nach Anspruch 12, bei der die genannten Haltemittel (40) Abschnitte der genannten aufrechtstehenden Bestandteile (38) umfassen.

14. Baugruppe nach Anspruch 11, bei der sich der genannte Kanal in Richtung auf das vordere Ende der Schnalle (14) befindet und die genannten aufrechtstehenden Bestandteile des genannten Eingriffmittels (38) sich hinter dem genannten Kanal befinden und Haltemittel (40) umfassen, die den genannten weiteren Abschnitt des Bandes halten und über dem genannten beschnittenen Ende liegen.

15. Baugruppe nach Anspruch 12, 13 oder 14, bei dem die genannten Haltemittel (40) an ihrer engsten Stelle über eine Distanz voneinander bestandet sind, die kürzer ist als die Breite des genannten weiteren Abschnitts des Bandes.

16. Baugruppe nach einem der Ansprüche 11 bis 15, bei der das genannte, mit der Fläche zusammenwirkende Mittel (34) mit der genannten Fläche (26) einen keilförmigen Kanal (Fig. 5) definiert, der zum vorderen Ende der Schnalle (14) hin enger wird.

17. Baugruppe nach einem der Ansprüche 11 bis 16, bei der das genannte Band (10) eine erste und eine zweite Öffnung in der Nähe seines genannten anderen Endes aufweist, und die genannte Schnalle (14) ein erstes (28) und ein zweites (30) Bestandteil umfaßt, die von der genannten Schnalle (14) wegverlaufen und jeweils in der genannten ersten und der zweiten Bandöffnung aufgenommen werden, um die Schnalle an dem Band (10) zu befestigen.

## Revendications

1. Assemblage pour le cerclage du sternum, utilisé avec une pince actionnée manuellement et comprenant :
- une bride souple oblongue (10) dont la première extrémité est dotée d'une aiguille (12) alors qu'une boucle est placée à proximité de la seconde, cette boucle présentant une surface (26) et un moyen (34) coopérant avec celle-ci pour définir un canal dans lequel la bride est susceptible d'être reçue,
- des moyens (36 et 37) pour bloquer une partie (24) de cette bride dans ce canal de manière à immobiliser cette dernière sous la forme d'une boucle de cerclage du sternum et
- un moyen (38) permettant l'engagement de la pince pour que celle-ci retienne la boucle (14) lorsque la partie (24) de la bride est immobilisée dans le canal par les moyens de blocage (36 et 37), caractérisé en ce que le moyen permettant l'engagement de la pince comprend deux éléments verticaux alignés par rapport au canal, chacun de ces éléments présentant une ouverture (41) destinée à recevoir une partie correspondante de la pince.

2. Assemblage selon la revendication 1, dans lequel le moyen permettant l'engagement de la pince (38) comprend des moyens (40) dans lesquels une partie supplémentaire de la bride est susceptible d'être retenue après avoir été repliée sur le moyen (34) coopérant avec la surface, la bride sortant de ce canal.

3. Assemblage selon la revendication 2, dans lequel les moyens de retenue (40) comprennent des parties des éléments verticaux (38).

4. Assemblage selon la revendication 1, dans lequel le canal est situé au niveau de l'extrémité avant de la boucle et les éléments verticaux du moyen (38) permettant l'engagement de la pince sont placés à l'arrière de ce canal et comprennent les moyens (40) destinés à retenir une partie supplémentaire de la bride (10) sortant du canal, ces moyens (40) ayant permis de replier la bande au-dessus du moyen (34) coopérant avec la surface.

5. Assemblage selon la revendication 2, 3 ou 4, dans lequel cette partie supplémentaire de la bride a une largeur donnée et dans lequel lesdits moyens de retenue (40) sont normalement espacés l'un de autre, en leur point le plus proche, d'une distance légèrement inférieure à cette largeur.

6. Assemblage selon l'une quelconque des revendications 1 à 5, dans lequel la bride comprend un élément (22) pour la guider dans le canal pendant la formation de la boucle.

7. Assemblage selon la revendication 6, dans lequel l'élément-guide (22) a la forme d'un dôme.

8. Assemblage selon la revendication 6, dans lequel le moyen (34) coopérant avec la surface (26) définit avec celle-ci un canal conique (figure 5) rétrécissant vers l'extrémité avant de la boucle (14).

9. Assemblage selon la revendication 6 ou 7, dans lequel la bride comprend une section (19) dont la largeur décroît au voisinage de l'élément-guide en forme de dôme (22) du côté dirigé vers la première extrémité de la bride (10).

10. Assemblage selon l'une quelconque des revendications précédentes, dans lequel la bride (10) possède des première et seconde ouvertures à proximité de la seconde extrémité, des premier (28) et second (30) éléments partant de la boucle (14) et étant respectivement reçus dans ces première et seconde ouverture pour fixer la boucle (14) à la bride.

11. Assemblage pour le cerclage du sternum, utilisé avec une pince actionnée manuellement et comprenant :
- une bride souple oblongue conçue pour permettre de réaliser une boucle (10), la première extrémité de cette bride étant effilée alors qu'une boucle (14) est placée à proximité de la seconde, cette boucle (14) présentant une surface (26) et un moyen (34) coopérant avec celle-ci pour définir un canal dans lequel une partie (24) de la bride (10) est reçue, la boucle ainsi formée partant de l'autre extrémité de cette partie (24) de la bride,
- des moyens (36 et 37) pour bloquer cette partie (24) de la bride dans le canal et
- un moyen (38) permettant l'engagement de la pince pour que celle-ci retienne la boucle (14) lorsque la partie (24) de la bride y est immobilisée par les moyens de blocage (36 et 37), caractérisé en ce que le moyen (38) permettant d'engager la pince comprend une paire d'éléments verticaux alignés par rapport au canal, chacun de ces éléments présentant une ouverture (41) destinée à recevoir une partie correspondante de la pince.

12. Assemblage selon la revendication 11, dans lequel ledit moyen permettant l'engagement de la pince (38) comprend des moyens (40) à l'intérieur desquels une partie supplémentaire de la bride comprenant l'extrémité effilée est retenue, cette partie supplémentaire et celle (24) de la bride immobilisée dans le canal étant reliées par une partie de bride (10) repliée au-dessus du moyen coopérant avec la surface.

13. Assemblage selon la revendication 12, dans lequel les moyens de retenue (40) comprennent des parties des éléments verticaux (38).

14. Assemblage selon la revendication 11, dans lequel le canal est dirigé vers l'extrémité avant de la bouche (14) et les éléments verticaux du moyen permettant l'engagement de la pince (38) sont situés à l'arrière de ce canal et comprennent des moyens (40) retenant ladite portion supplémentaire de bride et recouvrant l'extrémité effilée de celle-ci.

15. Assemblage selon la revendication 12, 13 ou 14, dans lequel les moyens de retenue (40) sont espacés l'un de l'autre, en leur point le plus proche, d'une distance inférieure à la largeur de la partie supplémentaire de bride.

16. Assemblage selon l'une quelconque des revendications 11 à 15, dans lequel le moyen (34) coopérant avec la surface (26) définit avec celle-ci un canal conique (figure 5) rétrécissant vers l'extrémité avant de la bouche (14).

17. Assemblage selon l'une quelconque des revendications 11 à 16, dans lequel la bride (10) possède des première et seconde ouvertures à proximité de sa première extrémité, des premier (28) et second (30) éléments partant de la bouche (14) et étant respectivement reçus dans ces première et seconde ouvertures pour fixer la bouche à la bride.
